# EUROPEAN PATENT APPLICATION

(11) **EP 3 998 331 A1**
(43) Date of publication of application: **18.05.2022**
(21) Application number: 20841387.2
(22) Date of filing: 08.07.2020
(51) Int. Cl.: C12M 1/34, G01N 1/10, G01N 1/40

(54) **SYSTEM FOR PREPARING SAMPLE SOLUTION FOR TESTING, AND METHOD FOR PREPARING SAMPLE SOLUTION FOR TESTING**

(30) Priority: 12.07.2019 JP 2019130254
(71) Applicant: IHI Corporation, Tokyo 135-8710 (JP)
(72) Inventor: IKEDA Ryousuke, Tokyo 135-8710 (JP); ISO Yoshiyuki, Tokyo 135-8710 (JP)
(74) Representative: TBK
(86) International application number: PCT/JP2020/026711
(87) International publication number: WO 2021/010256

(57) **Abstract**

A system for preparing a sample solution for testing includes a microorganism separation device, and prepares a sample solution for testing from an undiluted sample solution. The microorganism separation device includes a hydrodynamic separation device and a liquid feeding unit configured to supply the undiluted sample solution to the hydrodynamic separation device. The hydrodynamic separation device includes a curved flow channel having a rectangular cross-section, and is configured to separate a liquid into a first segment and a second segment through use of a vortex flow generated in the liquid flowing through the curved flow channel. Particles contained in the undiluted sample solution are concentrated in the first segment.

## Description

### TECHNICAL FIELD

The present disclosure relates to a system for preparing a sample solution for testing and a method for preparing a sample solution for testing in order to obtain, from an undiluted sample solution, a sample solution for testing in a suitable state for testing for the purpose of testing of microorganisms or the like contained in a liquid.

### BACKGROUND ART

In general, a microorganism test is conducted in order to control safety and quality of products while pharmaceutical products, and food and drink are manufactured. In the test, contamination with fungi such as yeast and mold, and bacteria such as colon bacillus and Legionella bacteria is checked. It is required to subject a large number of samples to testing for sufficient and proper product management.

Hitherto, a culture method has been used for the microorganism test. A liquid sample is used to perform culture with an agar medium, and the number of colonies on the culture medium is counted as the number of living bacteria. However, the culture method requires a long time period for culture, and hence rapid counting is difficult to achieve. In view of this, for the purpose of achieving rapid counting of living bacteria, various counting methods have been developed in recent years. As one of those methods, there has been proposed a method for counting the number of living bacteria by optically analyzing particles dispersed in the fluid (see Non-patent Literature 1 given below).

Further, in a case of liquid pharmaceutical products such as an injection solution, a test is conducted for foreign matters and fine particles contained in the liquid. Japanese Pharmacopeia specifies that a test is conducted in accordance with a light-shutoff particle counting method or a microscope coefficient method. In a case of an expensive liquid such as an injection solution, a product loss during the test is undesirable. Thus, it is conceived to recover a liquid in which particles that may contained in a sample before testing are concentrated so as to suppress a product loss. However, during this operation, there may be caused a risk of damaging particles due to a centrifugal force or a shearing force applied to a sample and deteriorating components due to a turbulent flow or bubbles (see Non-patent Literature 2 given below).

As a literature with regard to separation of particles contained in a fluid, Patent Literature 1 is given below that describes a hydrodynamic separation device including a curved channel. In this device, a fluid containing particles is supplied to the curved channel, and the particles are separated through use of a force acting on the fluid flowing through the curved channel.

### CITATION LIST

### PATENT LITERATURE

Patent Literature 1: Japanese Unexamined Patent Application Publication (Translation of PCT Application) No. 2016-526479

### NON-PATENT LITERATURE

Non-patent Literature 1: Sasaki Yasuhiko, Takeuchi Ikuo, Watanabe Yusuke, and Inami Hisao, "Easy and Rapid Live Bacteria Counter by Flow Cytometry. Japan journal of food engineering", Vol. 14, 131-136, September 2013
Non-patent Literature 2: Yamaura, Kazuo, "Denaturation of Biopolymer by Flow and Shaking of Solution", Journal of The Society of Rheology, Vol. 11, 107-116, (1983)

### SUMMARY OF THE INVENTION

In the counting method as described in Non-patent Literature 1, for the purpose of removing contaminants being obstacles for measurement or concentrating bacterial bodies contained in a sample, preliminary processing is required. With this, efficient counting is achieved. For example, when solid particles and the like derived from raw materials are contained in a sample of food and drink, those substances need to be removed. During such preliminary processing, an impact on bacterial bodies is undesirable. Further, microorganism contamination during preliminary processing needs to be avoided. Therefore, it is desired that preliminary processing be performed in an aseptic state. When a sample for a foreign matter test is subjected to preliminary processing that damages particles or degrades components, correct testing results cannot be obtained, and there is a difficulty in recovering a liquid.

As described above, when pharmaceutical products, food and drink, and the like are manufactured, problems such as cost increase and efficiency reduction of a sample for a microorganism test or a foreign matter test need to be solved. Here, preliminary processing for a testing sample is a key factor. The separation technique described in Patent Literature 1 relates to separation of solid particles. Still, regarding the key factor, when a sample is obtained for the purpose of a microorganism test, an impact on microorganisms needs to be examined.

Therefore, it is important to prepare a sample enables an efficient test. When the separation technique is applied to a test for management of product manufacturing, sufficient examination on whether a proper sample can be obtained is required.

It is an object of the present disclosure to provide a system for preparing a sample solution for testing and a method for preparing a sample solution for testing that enable an efficient test and are capable of suppling a sample solution for testing without affecting microorganisms.

### SOLUTION TO PROBLEM

In order to achieve the above-mentioned object, there has been examined an impact on microorganisms, which is caused at the time of performing separation of particle-like microorganisms or solid matters dispersed in an undiluted sample solution. As a result, it has been found that a suitable sample solution for testing can be obtained by separating and concentrating microorganisms or particle-like solid matters contained in an undiluted sample solution efficiently in accordance with a particle size and concentrating through use of a hydrodynamic separation technique.

According to one aspect of the present disclosure, a system for preparing a sample solution for testing is configured to prepare a sample solution for testing from an undiluted sample solution, and includes a microorganism separation device. The microorganism separation device includes a hydrodynamic separation device and a liquid feeding unit. The hydrodynamic separation device includes a curved flow channel having a rectangular cross-section, and is configured to separate a liquid into a first segment and a second segment through use of a vortex flow generated in the liquid flowing through the curved flow channel. The liquid feeding unit is configured to supply the undiluted sample solution to the hydrodynamic separation device. With the hydrodynamic separation device, particles contained in the undiluted sample solution are concentrated in the first segment.

The undiluted sample solution may include a liquid raw material, a liquid intermediate, and a liquid final product in manufacturing food and drink or pharmaceutical products.

The liquid feeding unit may include a supply channel being branched from a manufacturing line and connected to the hydrodynamic separation device, and a pump configured to apply, to the undiluted sample solution, a hydrodynamic pressure for supplying the undiluted sample solution to the hydrodynamic separation device. It may be configured that part of a liquid raw material, a liquid intermediate, or a liquid final product that flows through a manufacturing line is supplied as the undiluted sample solution to the hydrodynamic separation device. The liquid feeding unit may further include a return channel that returns one of the first segment and the second segment to the manufacturing line. Alternatively, the liquid feeding unit may include a container configured to store the undiluted sample solution, a supply channel configured to connect the container to the hydrodynamic separation device, and a pump configured to apply, to the undiluted sample solution, a hydrodynamic pressure for supplying the undiluted sample solution to the hydrodynamic separation device. Further, it is suitable that the liquid feeding unit further includes a return channel configured to return one of the first segment and the second segment to the container. The liquid feeding unit may further include a circulation channel configured to cause a part of another one of the first segment and the second segment to circulate to the supply channel.

The system for preparing a sample solution for testing may further include an additional hydrodynamic separation device. It may be configured that the liquid feeding unit further includes a flow channel for supplying, to the additional hydrodynamic separation device, a remaining part of another one of the first segment and the second segment after separation in the hydrodynamic separation device. Further, it may be configured that the system for preparing a sample solution for testing further includes a test device, a flow channel configured to connect the hydrodynamic separation device to the test device, and a flow channel configured to connect the test device to the manufacturing line. Another one of the first segment and the second segment may be returned to the manufacturing line via the test device.

Moreover, according to one aspect of the present disclosure, a method for preparing a sample solution for testing is performed to prepare a sample solution for testing from an undiluted sample solution, and includes microorganism separation processing. The microorganism separation processing includes a hydrodynamic separation step of separating a liquid into a first segment and a second segment through use of a vortex flow generated in the liquid flowing through a curved flow channel having a rectangular cross-section, and a liquid feeding step of feeding the undiluted sample solution to the hydrodynamic separation step. In the hydrodynamic separation step, particles contained in the undiluted sample solution are concentrated in the first segment.

The particles may be microorganisms of at least one type selected from a virus, a eubacterium, an archaebacterium, a fungus, a myxomycete, an alga, and a protist, the first segment may be supplied as a sample solution for testing, and the return channel may return the second segment from the hydrodynamic separation device. Further, the particles may be particle-like solid matters larger than microorganisms, and the second segment may be supplied as a sample solution for testing. At this state, the first segment may be returned through the return channel, or may be discharged to the outside of the system.

### ADVANTAGEOUS EFFECTS

According to the present disclosure, the particle-like microorganisms or the particle-like solid matters dispersed in the liquid can be separated efficiently. At the same time, the liquid after separation can be used as the sample solution for testing, and a test for microorganisms that may be contained in the undiluted sample solution can be conducted efficiently. Further, a separation operation can be performed in a sequential and aseptic manner, and hence contamination can be prevented during preparation for the sample solution for testing. Therefore, there can be provided the system for preparing a sample solution for testing and the method for preparing a sample solution for testing that enable efficient preparation for the sample solution for testing, and efficient control for safety and quality of products can be performed.

### BRIEF DESCRIPTION OF DRAWINGS

[FIG. 1] FIG. 1 is a schematic configuration diagram illustrating a system for preparing a sample solution for testing according to a first embodiment.
[FIG. 2] FIG. 2 is a schematic configuration diagram illustrating a system for preparing a sample solution for testing according to a second embodiment.
[FIG. 3] FIG. 3 is a schematic configuration diagram illustrating a system for preparing a sample solution for testing according to a third embodiment.
[FIG. 4] FIG. 4 is a schematic configuration diagram illustrating a system for preparing a sample solution for testing according to a fourth embodiment.
[FIG. 5] FIG. 5 is a schematic configuration diagram illustrating a system for preparing a sample solution for testing according to a fifth embodiment.
[FIG. 6] FIG. 6 is a schematic configuration diagram illustrating a system for preparing a sample solution for testing according to a sixth embodiment.
[FIG. 7] FIG. 7 is a schematic configuration diagram illustrating a system for preparing a sample solution for testing according to a seventh embodiment.
[FIG. 8] FIG. 8 is a graph showing a relationship between a De number and separation efficiency in cell separation performed by a hydrodynamic separation device.
[FIG. 9] FIG. 9 is a graph showing a relationship between a pump used for cell separation and a cell survival rate.

### DESCRIPTION OF EMBODIMENTS

Embodiments of the present disclosure are described below in detail with reference to the drawings. Note that dimensions, materials, and other specific numerals in the embodiments are given for easy understanding of the contents, and do not limit the present disclosure unless otherwise noted. Further, in description and the drawings of the present application, elements having substantially the same function and configuration are denoted with the same reference symbol, and redundant description therefor is omitted. Elements that are not directly related to the present disclosure are omitted in illustration.

When food and drink or pharmaceutical products are produced, a microorganism test, a foreign matter test, or the like is conducted for the purpose of controlling safety and quality of the products. In a microorganism test, the number of microorganisms contained in a sample solution is counted, thereby confirming microorganism contamination. The number of microorganisms is counted by optically analyzing particles dispersed in a fluid. With this, the number of living bacteria can be counted rapidly. For such efficient counting, it is effective to prepare a sample solution in which microorganisms that may be contained in an undiluted sample solution are concentrated. Further, when particle-like solid matters larger than the microorganisms are contained in the undiluted sample solution, a sample solution for testing from which such matters are removed as contaminants is prepared, which is effective for precise counting. Further, in a foreign matter test, when a sample solution in which particles that may be contained in a liquid are concentrated is prepared, the remaining part of the undiluted sample solution can be recovered. Thus, when no abnormality is found during a foreign matter test, the recovered solution can be reused. Therefore, concentration of particles contained in a liquid and separation in accordance with a size are key factors in preparing a sample solution for testing.

One of the separation techniques for separating particles contained in a liquid uses an effect of a Dean vortex generated in the liquid (see Patent Literature 1 given above; hereinafter, the technique is referred to as hydrodynamic separation). This separation technique utilizes uneven distribution of the particles in the liquid in which a Dean vortex is generated. The liquid flows through a curved flow channel that has a rectangular cross-section vertical to a flow direction and is curved to one side. The particles flowing through the curved flow channel are distributed differently in the flow channel in accordance with a size (see Patent Literature 1 given above). Specifically, ring-like particle distribution is formed in the cross-section of the flow channel, and the particles spirally flow through the flow channel. In this state, a particle having a relatively large size is positioned on the outer side of the ring, and a particle having a relatively small size is positioned on the inner side of the ring. Further, when a predetermined separation condition is set, a particle distribution mode further changes. As a result, particles having a size exceeding a certain level converge on the outer circumferential side of the flow channel.

In the present disclosure, the above-mentioned hydrodynamic separation is applied to separation of particles, in other words, microorganisms or solid matters having a particle-like shape contained in the liquid, and is used in a separation mode in which microorganism particles or solid matter particles having a predetermined size or larger converge on the outer circumferential side of the curved flow channel. With this, a liquid raw material, a liquid intermediate, or a liquid final product in manufacturing products is used as an undiluted sample solution, and then a sample solution for testing is prepared. In the system for preparing a sample solution for testing according to the present disclosure, a liquid is supplied to a curved flow channel at a relatively high flow speed. As described above, while the liquid to be supplied flows through the curved flow channel, relatively small particles are distributed in a ring shape in the cross-section of the flow channel. Meanwhile, relatively large particles converge evenly on the outer side (outer circumferential side) of the curved flow channel. In other words, a liquid in which the relatively large particles are concentrated can be isolated as a first segment. Therefore, when the undiluted sample solution contains microorganism particles or solid matter particles, the undiluted sample solution is separated into the first segment in which the particles are concentrated and a second segment being the rest. With this, the first segment can be used as a sample solution for a microorganism test or a foreign matter test. When there is no abnormality found during the test, the second segment being the rest can be recovered and reused. With this, in a case of an undiluted sample solution in manufacturing an expensive pharmaceutical product such as an injection solution, a loss caused as a result of testing can be reduced. The microorganisms are separated in accordance with a particle size during hydrodynamic separation. Thus, the microorganisms are separated as particle-like organisms, and may be any one of microorganism single cells and particle-like aggregates of a plurality of cells. The solid matter particles may be any one of organic particles and inorganic particles, and the solid matter particles formed of, for example, plastic, elastomer, fibers, ceramics, and metal can be separated suitably.

An undiluted sample solution in manufacturing food and drink contains particle-like solid matters larger than microorganisms being a test target in some cases. Specifically, the particles contained in the undiluted sample solution may contain relatively large particle-like solid matters and relatively small microorganism particles. For example, when a seasoning liquid such as soy sauce is used as a liquid raw material, fine particles derived from the raw material or component aggregates may be contained. The same holds true to a liquid intermediate in the course of manufacture. Tea beverage, fruit beverage, or the like being a liquid final product contains particle-like solid matters such as tea leaf particles, fruit particles, or the like. In this case, during hydrodynamic separation, the particle-like solid matters being relatively large particles are concentrated in the first segment, and the second segment being the rest contains most of the relatively small particles. Therefore, the second segment obtained after removing the particle-like solid matters is used as a sample solution for testing in a microorganism test. With this, microorganism particles being relatively small particles can be detected. When there is no abnormality found during the test, the first segment may be recovered and reused. When the undiluted sample solution contain microorganisms, the second segment in this case may contain dead cell pieces (debris) and the like. In order to remove debris and the like from the sample solution for testing, a separation state may be adjusted, and hydrodynamic separation is further repeated. With this, microorganisms being relatively large particles and debris being relatively small particles can be separated. A size of the concentrated particles can be adjusted based on settings of a flow speed (flow rate) of the liquid supplied to hydrodynamic separation and a size of the cross-section of the curved flow channel.

A microorganism grows large in a highly active state. However, an active level is lowered, a microorganism is dead and dissolved while having a relatively small size. Most of the relatively small microorganism particles are dead microorganisms or dead microorganism pieces, and the chances that active cells in the course of DNA synthesis are contained are low. A size of the particles to be separated through hydrodynamic separation can be adjusted by changing a separation condition (a supply flow rate of the liquid). Thus, by repeating hydrodynamic separation under different separation conditions, active microorganisms and debris and the like can be separated. Through multi-step hydrodynamic separation in which hydrodynamic separation is repeated while changing the separation condition, particles having different particle sizes can be isolated at the respective steps. Alternatively, when hydrodynamic separation is repeated under a constant separation condition, a concentration degree and separation accuracy can be improved.

In hydrodynamic separation, a flow in the flow channel is a laminar flow. Relatively, fracture of the particles contained in the liquid is less likely to happen, which is significantly effective in preparing a sample solution for a foreign matter test. However, considering a continuous and efficient microorganism test and an action performed when culture is required, presence or absence of damage on microorganisms during preparation for a sample solution needs to be examined. As a result of examination on this point, microorganisms such as a yeast and fungi have resistance under a relatively high pressure, and a survival rate thereof is maintained even when a pressure of, for example, approximately 1 MPa is applied. Moreover, the microorganisms have considerably high durability against pressure fluctuation. In a case where animal cells are cultured, the animal cells are affected by fluctuation of a pressure (pressure reduction) applied to the cell during separation, and a survival rate of the cells is lowered easily. Nevertheless, even when the microorganisms are under considerably large pressure fluctuation during hydrodynamic separation, a survival rate thereof can be maintained. Therefore, during hydrodynamic separation for microorganism particles, it is not particularly required to control a pressure environment. Thus, a hydrodynamic pressure can be changed by changing a driving force of a pump or the like that is used to supply a liquid to a hydrodynamic separation device, and hence a flow rate of the liquid can be adjusted based on a hydrodynamic pressure in a freely selective manner. In other words, settings for the separation condition can be changed easily through drive control of a pump, and hence the system configuration can be easily simplified. Further, hydrodynamic separation has advantages in that an aseptic state of the liquid can be easily maintained and that separation with high reproducibility can be achieved regardless of the skill level of an operator.

When an impact on microorganisms being a test target, which is caused by a pressure environment, is concerned, fluctuation of a pressure applied to microorganisms through hydrodynamic separation may be controlled. In this case, the separation condition is set so that fluctuation of a pressure (pressure reduction) applied to the cell during separation does not exceed a certain level. With this, a pressure environment in which the liquid is supplied to the curved flow channel is controlled. In other words, supply of the liquid is controlled so that fluctuation of a pressure (a difference between an inlet pressure and an outlet pressure) applied to the microorganisms during separation is equal to or lower than a predetermined value. Specifically, control is performed appropriately when pressure fluctuation (pressure difference) is set to be less than 0.60 MPa, preferably, equal to or less than 0.45 MPa, more preferably, equal to or less than 0.40 MPa. Under the separation condition for controlling pressure fluctuation as described above, microorganisms are less damaged, and a survival rate of the microorganisms can be maintained at approximately 98% or higher. Therefore, large microorganism particles after concentration and separation can be isolated, and a proliferation function or the like thereof can be maintained. Under this state, the large microorganism particles can be supplied for measurement of particle diameter distribution, microorganism culture, testing, and the like.

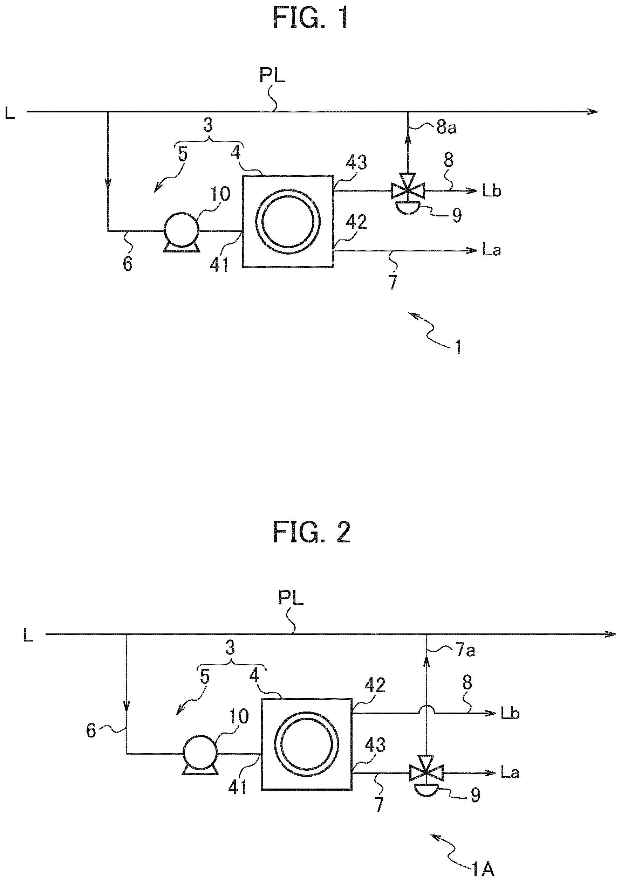

With reference to the embodiments illustrated in the drawings, the configuration of the system for preparing a sample solution for testing is described below. A system 1 for preparing a sample solution for testing in FIG. 1 includes a microorganism separation device 3. Through use of this device, a sample solution for testing is prepared from an undiluted sample solution L. The microorganism separation device 3 includes a hydrodynamic separation device 4 and a liquid feeding unit 5 that supplies the undiluted sample solution L to the hydrodynamic separation device 4. The hydrodynamic separation device 4 includes a curved flow channel having a rectangular cross-section. Through use of a vortex flow generated by the liquid flowing through the curved flow channel, the liquid is separated into the first segment and the second segment. With The hydrodynamic separation device 4, particles contained in the undiluted sample solution are concentrated in the first segment.

In the embodiment of FIG. 1, the undiluted sample solution L is isolated from a manufacturing line PL. Specifically, the liquid feeding unit 5 includes a supply channel 6 and a pump 10. The supply channel 6 includes a pipe that is branched from the manufacturing line PL and is connected to the hydrodynamic separation device 4. The pump 10 applies, to the undiluted sample solution L, a hydrodynamic pressure for supplying the undiluted sample solution L to the hydrodynamic separation device 4. Part of a liquid raw material, a liquid intermediate, or a liquid final product that flows through the manufacturing line PL is supplied as the undiluted sample solution L to the hydrodynamic separation device 4.

The hydrodynamic separation device 4 includes the curved flow channel inside, which has a constant rectangular cross-section vertical to the flow direction. At one end of the curved flow channel, a single inlet port 41 through which the undiluted sample solution L is taken in is formed. At the other end of the curved flow channel, at least two outlet ports 42 and 43 for separating and discharging the undiluted sample solution are formed. While the liquid flows through the curved flow channel, a vortex flow is generated in the liquid swirling in one direction. The hydrodynamic separation device 4 utilizes the vortex flow, and causes relatively large particles among the particles contained in the undiluted sample solution L to be evenly present on the outer side (outer circumferential side) of the flow channel. Therefore, the liquid discharged from the curved flow channel is divided into the outer-side segment and the inner-side segment, and hence the liquid in which the relatively large particles are concentrated can be separated as the outer-side segment. A liquid La (first segment) containing the concentrated particles having a relatively large size is discharged from the one outlet port 42 through a flow channel 7 connected to the outlet port 42. A liquid Lb (second segment) being the rest that contains relatively small microorganism particles is discharged through a flow channel 8 connected to the other outlet port 43.

Therefore, when the undiluted sample solution L containing particles that are not larger than the microorganisms being a test target, the microorganism particles are concentrated in the first segment, and can be obtained as the liquid La from the outlet port 42. The liquid La can be used as a sample solution for testing in a microorganism test, and thus presence or absence of microorganisms can be tested. As required, counting of the number of microorganism particles, measurement of particle diameter distribution and a particle shape, and the like may be performed.

The curved shape of the curved flow channel may be a substantially circular shape, a substantially arc shape (partial circumference), a spiral shape, or the like, and may be selected freely from those shapes. The hydrodynamic separation device 4 may be designed with a flow channel unit including one curved flow channel as a flow channel unit. Specifically, the flow channel unit is configured in the following manner. A molded body having a plane layer is formed of plastic or the like, and one curved flow channel is formed inside the molded body. Then, the end surfaces of the molded body are opened at both the terminal ends of the curved flow channel, and hence one inlet port and at least two outlet ports are formed. The molded body as described above is used as the flow channel unit. The hydrodynamic separation device may consist of one flow channel unit or a combination of a plurality of flow channel units. When the plurality of flow channel units are layered to form the flow channel in a parallel state, a processing flow rate of the undiluted sample solution L can be increased.

Efficiency in separating the particles in the hydrodynamic separation device 4 is changed in accordance with a De number and a pressure of the liquid supplied to the curved flow channel. When the De number and the pressure fall within proper ranges, separation can be performed suitably. The Dean number is expressed in Equation of De=Re(D/2Rc)^{1/2} (Re denotes a Reynold's number (-), D denotes a representative length (m), and Rc denotes a turning radius of the flow channel (m)). The De number is proportional to a flow speed of the liquid. Thus, when a flow speed of the liquid supplied to the hydrodynamic separation device is controlled properly, the particles can be separated suitably. In general, the De number is preferably 30 or greater and 100 or less, more preferably, approximately from 50 to 80. Therefore, a flow speed (flow rate) of the liquid is set so that the De number falls within this range. In accordance with a size of the particles to be separated, a flow rate of the liquid supplied to the hydrodynamic separation device 4 may be adjusted.

The liquid feeding unit 5 of the system 1 for preparing a sample solution for testing includes a pressurizing device that applies, to the undiluted sample solution L, a hydrodynamic pressure for supplying the undiluted sample solution L to the hydrodynamic separation device 4, namely, the pump 10. The hydrodynamic pressure applied by the flow the pump 10 changes a flow rate and a flow pressure at which the undiluted sample solution L is supplied to the hydrodynamic separation device 4. A survival rate of the microorganisms is an important factor when a microorganism test is conducted. In this regard, the microorganisms have durability against separation performed by the hydrodynamic separation device 4, and a survival rate can be maintained. However, in a case where it is desired that an impact of pressure fluctuation be eliminated, a suitable pressure environment can be controlled through drive control of the pump 10, for example. When a pressure environment is controlled so that a pressure difference is less than 0.60 MPa, preferably, equal to or less than 0.45 MPa, more preferably, equal to or less than 0.40 MPa, reduction of a survival rate and damage on the microorganisms during separation can be prevented. When a pressure control valve and a flow rate adjusting valve are provided, a supply pressure and a supply flow rate of the undiluted sample solution can be adjusted in a freely selective manner. Thus, a pressure and a flow rate may be monitored through use of a pressure gauge and a flowmeter.

In the system 1 for preparing a sample solution for testing in FIG. 1, drive control of the pump 10 is utilized in place of the flow rate adjusting valve so that the flow rate of the liquid supplied to the hydrodynamic separation device 4 is adjusted properly. A supply flow rate and a pressure of the liquid can be regulated to some extent by designing dimensions of the supply channel 6 and the flow channels 7 and 8 properly based on processing capacity (a size of the cross-section of the flow channel and the number of flow channels) of the hydrodynamic separation device 4. Therefore, even with a simple configuration as in the system 1 for preparing a sample solution for testing in FIG. 1, the sample for testing can be efficiently and continuously prepared through particle separation corresponding to a microorganism test.

When an impact on the microorganisms is eliminated as much as possible while preparing a sample solution for testing, a pump of such a type that does not apply a shearing force to the microorganisms may be used. Specifically, it is suitable to use a positive displacement pump that utilizes a volume change caused by reciprocating motion or rotating motion and push out a liquid having a predetermined volume. Examples of the positive displacement pump include a reciprocating pump such as a piston pump, a plunger pump, a diaphragm pump, and a wing pump, and a rotary pump such as a gear pump, a vane pump, and a screw pump. In one embodiment, for example, a pressure tank to which a compressor is attached may be used. In this embodiment, a liquid stored in the pressure tank can be pressurized by the compressor, and then the liquid can be forcibly fed from the pressure tank to the hydrodynamic separation device.

The liquid feeding unit of the system for preparing a sample solution for testing may further include a return channel that returns one of the first segment and the second segment to the manufacturing line. The system 1 for preparing a sample solution for testing in FIG. 1 includes a return channel 8a that is branched from the flow channel 8 and is connected to the manufacturing line. The return channel 8a is configured so that the liquid Lb being the rest discharged as the second segment from the outlet port 43 can be returned to the manufacturing line. A switching valve 9 is provided at a branching point of the return channel 8a, and is capable of selecting any one of discharging to the outside of the system and returning to the manufacturing line with respect to the second segment. Therefore, recovery through use of the switching valve can reduce a loss caused as a result of testing. When the first segment contain microorganisms, the second segment isolated through hydrodynamic separation may contain relatively small microorganism cells, debris, and the like in some cases. Thus, the second segment from which abnormality is found during testing may be disposed. When an expensive component or a rear substance is contained in the undiluted sample solution, a filter may be used to remove debris or aggregates. Then, refinement processing may be performed to recover a desired component. A filter may be selected with an appropriate hole diameter according to an object to be removed. Examples of the filter include a precision filtration film and an ultra-filtration film.

In the system 1 for preparing a sample solution for testing, a method for preparing a sample solution for testing is performed to prepare a sample solution for testing from an undiluted sample solution. The preparation method involves microorganism separation processing. The microorganism separation processing includes a hydrodynamic separation step and a liquid feeding step of supplying the undiluted sample solution to the hydrodynamic separation step. In the hydrodynamic separation step, the liquid is separated into the first segment and the second segment through use of a vortex flow generated in the liquid flowing through the curved flow channel having a rectangular cross-section. With this, the particles contained in the undiluted sample solution are concentrated in the first segment. An undiluted sample solution containing particles that are not larger than the microorganisms being a test target. With this, the liquid in which the microorganisms are concentrated can be obtained as the first segment. This liquid can be used as a sample solution for testing in a microorganism test, thereby confirming presence or absence of microorganisms. As required, counting of the number of microorganism particles, measurement of particle diameter distribution and a particle shape, and the like may be performed in combination.

In other words, the above-mentioned particles being the separation target during hydrodynamic separation are microorganism particles. The first segment is supplied as a sample solution for testing. The second segment is discharged from the hydrodynamic separation device to the outside of the system, or is returned to the manufacturing line PL through the return channel. The microorganism particles having a size of approximately 0.1 µm to a few millimeters may be suitably separated. Microorganism particles exceeding such a size can be separated in accordance with the separation condition. Microorganisms of at least one type selected from a virus, a eubacterium, an archaebacterium, a fungus, a myxomycete, an alga, and a protist may be used as a test target.

In the hydrodynamic separation step, the undiluted sample solution is introduced through a single inlet port into the curved flow channel having a rectangular cross-section, and is supplied to the curved flow channel under a uniformed state. The curved flow channel of the hydrodynamic separation device is a flow channel having a rectangular cross-section vertical to the flow direction (radial cross-section). While the uniform liquid flows through the curved flow channel, distribution is changed to a ring-like shape in the rectangular cross-section with a Dean vortex. At the same time, a dynamic lifting force stagnating on the outer circumferential side of the flow channel acts on the particles, and distribution thereof is concentrated on the outer circumferential side. The terminal end outlet of the curved flow channel is divided into two ports, namely, the outlet port 42 positioned on the outer circumferential side and the outlet port 43 positioned on the inner circumferential side. The liquid containing the concentrated particles is discharged from the outlet port 42 on the outer circumferential side, and the liquid being the rest is discharged from the outlet port 43 on the inner circumferential side. The separation condition in the hydrodynamic separation step can be changed by adjusting a flow rate in the liquid feeding step. An appropriate flow rate may be set in accordance with a size of the particles being the separation target.

A system 1A for preparing a sample solution for testing in FIG. 2 corresponds to one example obtained by modifying the system for preparing a sample solution for testing in FIG. 1 so that the second segment obtained through hydrodynamic separation is supplied as a sample solution for testing. Therefore, a return channel 7a that returns the liquid to the manufacturing line PL is configured to be branched from the flow channel 7 for discharging the first segment and be connected to the manufacturing line PL. Similarly, the switching valve 9 is provided at a branching point of the return channel 7a, and is capable of selecting any one of discharging to the outside of the system and returning to the manufacturing line PL.

In FIG. 1, the particles that may be contained in the undiluted sample solution are microorganism particles. In contrast, in the method for preparing a sample solution for testing, which is performed in the system 1A for preparing a sample solution for testing in FIG. 2, an undiluted sample solution containing particle-like solid matters larger than microorganisms is used. In other words, the particles being the separation target during hydrodynamic separation are particle-like solid matters larger than the microorganisms, and are separated as the first segment. The first segment is returned through the return channel 7a, or is discharged to the outside of the system. For example, when the particle-like solid matters are contaminants, waste, or the like being obstacles for testing, the liquid La containing the particle-like solid matters is discharged through the flow channel 7. When the particle-like solid matters are components allowed to be in a product such as raw material particles of food and drink, the particle-like solid matters may be returned to the manufacturing line PL. When the undiluted sample solution contains microorganisms, most of the microorganisms being relatively small particles are contained in the second segment. Therefore, when the second segment from which the particle-like solid matters are removed is used as a sample solution for testing, the microorganisms are detected.

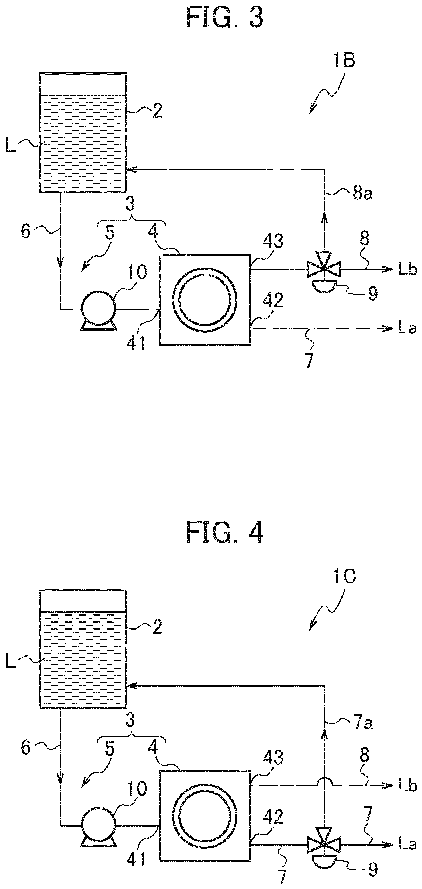

A system 1B for preparing a sample solution for testing in FIG. 3 corresponds to one example obtained by modifying the system for preparing a sample solution for testing in FIG. 1 so that the undiluted sample solution stored in a container is used to prepare a sample solution for testing. Specifically, the liquid feeding unit 5 of the system 1B for preparing a sample solution for testing includes a container 2 that stores the undiluted sample solution L, the supply channel 6 that connects the container 2 to the hydrodynamic separation device 4, and the pump 10 that applies, to the undiluted sample solution, a hydrodynamic pressure for supplying the undiluted sample solution L to the hydrodynamic separation device 4. When the undiluted sample solution L contains particles that are not larger than the microorganisms being a test target, the microorganism particles are concentrated in the first segment, and are obtained as the liquid La through the outlet port 42 and the flow channel 7. Therefore, the liquid La is used as a sample solution for testing in a microorganism test, thereby confirming presence or absence of microorganisms. As required, counting of the number of microorganism particles, measurement of particle diameter distribution and a particle shape, and the like may be performed.

The liquid feeding unit 5 of the system 1B for preparing a sample solution for testing may further include a return channel that returns one of the first segment and the second segment to the container 2. In the embodiment in FIG. 3, the return channel 8a branched from the flow channel 8 is connected to the container 2. In other words, the configuration can be made so that the liquid Lb being the rest, which is discharged as the second segment from the outlet port 43, can be returned to the container 2. The switching valve 9 is provided at the branching point of the return channel 8a, and is capable of selecting any one of discharging to the outside of the system or returning to the container 2 with respect to the second segment. Therefore, the second segment can be recovered and reused, which can be utilized to reduce a loss caused as a result of testing.

The container 2 is a container capable of preventing microorganism contamination. A container equipped with a heater or a cooler and having a temperature adjusting function may be used as the container 2, as required. The liquid inside is maintained at a temperature suitable for storing the undiluted sample solution. The container 2 may include a stirring device for uniformizing the liquid. With this, stirring can be performed at such an appropriate speed that the microorganisms are not damaged. Alternatively, in order to perform adjustment to achieve an environment suitable for the microorganisms, a container having a function of adjusting an amount of oxygen/carbon dioxide/air, pH, conductivity, a light amount, or the like may be used as required.

The method for preparing a sample solution for testing that can be performed in the system 1B for preparing a sample solution for testing in FIG. 3 is similar to that performed in the system 1 for preparing a sample solution for testing in FIG. 1, except that the second segment is not returned to the manufacturing line PL but to the container 2. In other words, the undiluted sample solution containing particles that are not larger than the microorganisms being a test target is used. When microorganisms are present, the particles are concentrated in the first segment. Therefore, the first segment can be used as a sample solution for testing in a microorganism test, thereby confirming presence or absence of microorganisms. As required, counting of the number of microorganism particles, measurement of particle diameter distribution and a particle shape, and the like may be performed. Microorganisms of at least one type selected from a virus, a eubacterium, an archaebacterium, a fungus, a myxomycete, an alga, and a protist may be used as a test target. The second segment is discharged from hydrodynamic separation device to the outside of the system, or is returned to the container 2 through the return channel. When significantly small particles such as a virus are the test target, a flocculation agent may be added to the undiluted sample solution in the container 2, and the particles may be aggregated to have a size that is readily subjected to hydrodynamic separation. In this case, the obtained first segment in which the aggregation is canceled may be supplied as a sample solution for testing.

The configuration in which the undiluted sample solution L is supplied from the container 2 is suitable to a case where the liquid that is directly extracted from the manufacturing line is subjected to dilution or filtration/separation, or the like and is used. Further, the liquid may be used at the time of conducting a test such as a control test using cultured microorganism, and a sample solution may be prepared.

A system 1C for preparing a sample solution for testing in FIG. 4 corresponds to one example obtained by modifying the system for preparing a sample solution for testing in FIG. 3 so that the second segment obtained through hydrodynamic separation is supplied as a sample solution for testing. Therefore, the return channel 7a that returns the liquid to the container 2 is configured to be branched from the flow channel 7 for discharging the first segment and be connected to the container 2. Similarly, the switching valve 9 is provided at the branching point of the return channel 7a, and is capable of selecting any one of discharging to the outside of the system and returning to the manufacturing line PL.

The method for preparing a sample solution for testing, which is performed in the system 1C for preparing a sample solution for testing in FIG. 4, is similar to that performed in the system 1B for preparing a sample solution for testing in FIG. 2. An undiluted sample solution containing particle-like solid matters larger than the microorganisms being a test target is used. Therefore, the particles being the separation target during hydrodynamic separation are relatively large particles, in other words, particle-like solid matters larger than the microorganisms, and are separated as the first segment. The first segment is returned from the outlet port 42 through the return channel 7a, or is discharged to the outside of the system. For example, when the particle-like solid matters are contaminants, waste, or the like being obstacles for testing, the liquid La containing the particle-like solid matters is discharged through the flow channel 7. In this case, a flocculation agent may be added to the undiluted sample solution in the container 2, and the particle-like solid matters may be aggregated. With this, hydrodynamic separation is facilitated. Alternatively, when the particle-like solid matters are components allowed to be in a product such as raw material particles of food and drink, the particle-like solid matters may be returned to the container 2. Most of the microorganisms being relatively small particles are contained in the second segment. Thus, the second segment discharged from the outlet port 43 of the hydrodynamic separation device 4 through the flow channel 8 is supplied as a sample solution for testing.

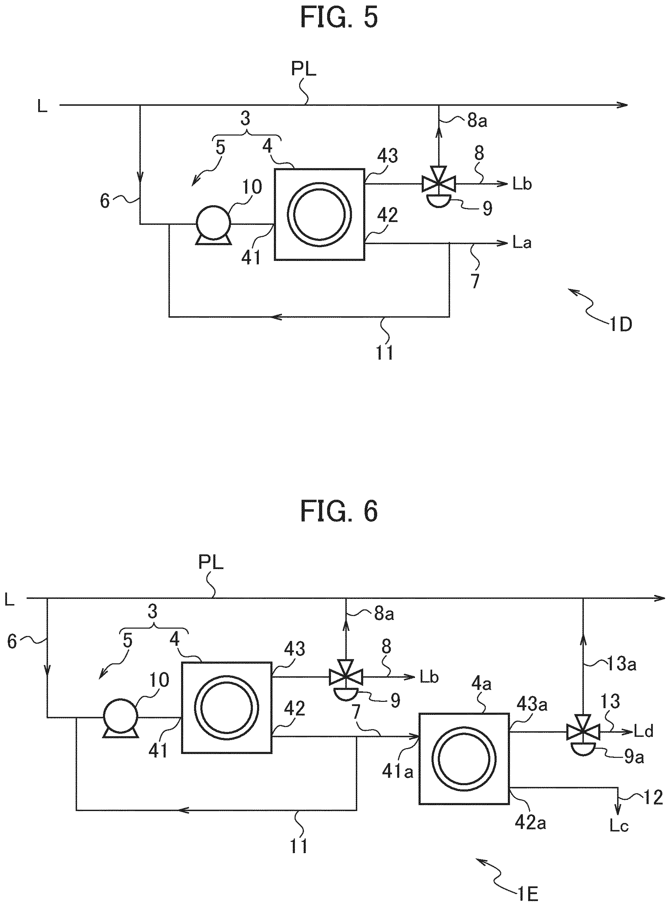

In the system 1 for preparing a sample solution for testing in FIG. 1, one of the first segment and the second segment (that is, the second segment) can be returned to the manufacturing line PL, and the other one is recovered as a sample solution for testing as it is. In contrast, a system 1D for preparing a sample solution for testing in FIG. 5 corresponds to one example in which a circulation channel 11 is further provided. The circulation channel 11 circulates the other one of the first segment and the second segment (that is, the first segment) to the supply channel 6. The circulation channel 11 is branched from the flow channel 7, and is connected to the supply channel 6 on the upstream side of the pump 10. Therefore, a part of the first segment is returned to the supply channel 6, and is subjected to hydrodynamic separation again. Thus, concentration of the particles in the first segment discharged from the flow channel 7 is gradually increased. In other words, a concentration degree of the particles can be improved. Therefore, when a significantly small number of particles is contained in the undiluted sample solution, and there is a difficulty in discriminating the particles from a measurement error in a test, measurement can be facilitated by improving the concentration degree. In other words, the concentration degree contributes to improvement of detection sensitivity in a test. The configuration of the system 1D for preparing a sample solution for testing in FIG. 5 may be applied to the system 1B for preparing a sample solution for testing in FIG. 3. The first segment obtained from the undiluted sample solution stored in the container is partially circulated, and thus the concentration degree can be improved.

A system 1E for preparing a sample solution for testing in FIG, 6 corresponds to one example obtained by modifying the system 1D for preparing a sample solution for testing in FIG. 5 so that an additional hydrodynamic separation device 4a is provided. In other words, the configuration is made so that two-step hydrodynamic separation can be performed. In FIG. 5, a part of the other one of the first segment and the second segment (that is, the first segment) is circulated to the supply channel 6. The remaining part thereof is supplied as a sample solution for testing as it is. In contrast, in FIG. 6, the flow channel 7 is connected to the additional hydrodynamic separation device 4a, and the remaining part of the first segment is supplied to the additional hydrodynamic separation device. In other words, the additional hydrodynamic separation device 4a is used to perform two-step hydrodynamic separation, which is advantageous in improving a concentration degree and separation accuracy of the particles in the first segment. A liquid Lc obtained as the first segment from an outlet port 42a of the hydrodynamic separation device 4a is supplied through a flow channel 12 as a sample solution for testing.

In the system 1E for preparing a sample solution for testing, a liquid Ld is discharged as the second segment through a flow channel 13 connected to an outlet port 43a of the hydrodynamic separation device 4a. Further, the configuration is made so that a return channel 13a that is branched from the flow channel 13 and is connected to the manufacturing line PL is provided. With this, the liquid Ld being the second segment can be returned to the manufacturing line PL. Similarly, a switching valve 9a is provided at a branching point of the return channel 13a, and is capable of selecting any one of discharging to the outside of the system and returning to the manufacturing line PL. The configuration of the system 1E for preparing a sample solution for testing in FIG, 6 may be applied to the system 1B for preparing a sample solution for testing in FIG. 3. With this, the first segment obtained from the undiluted sample solution stored in the container may be subjected to multi-step hydrodynamic separation.

In the system 1E for preparing a sample solution for testing in FIG, 6, two-step separation is performed, and hence components contained in the liquid are divided into three groups. A separation condition for the hydrodynamic separation device 4a for the second step can be adjusted by controlling a supply flow rate of the liquid La supplied through the flow channel 7. Thus, a pump may be provided to the flow channel 7, and the separation condition may be adjusted. The separation conditions in the two hydrodynamic separation devices 4 and 4a are set appropriately. With this, separation accuracy and a concentration degree of the particles can be improved. Note that, in accordance with a division ratio of the two segments in the hydrodynamic separation device for the first step, processing capacity of the hydrodynamic separation device for the second step is set appropriately.

Note that multi-step hydrodynamic separation as performed in the system 1E for preparing a sample solution for testing in FIG, 6 can be performed through use of the system 1D for preparing a sample solution for testing in FIG. 5. In this case, the configuration is made so that a switching valve is provided at each of a branching point and circulating point of the circulation channel 11. With this, the entirety of the first segment can be switched to one of discharging to the outside of the system and circulation. In this case, a container for temporary storage may be provided to the circulation channel 11. The first segment discharged from the hydrodynamic separation device 4 is stored in the container, the separation condition for hydrodynamic separation is changed, and the first segment is supplied to the hydrodynamic separation device 4 again. With this, two-step hydrodynamic separation can be performed. The separation condition can be changed by, for example, adjusting a driving force of a pump. In this manner, the separation condition is changed in a stepwise manner, and a separation operation is repeated. With this, multi-step separation can be performed.

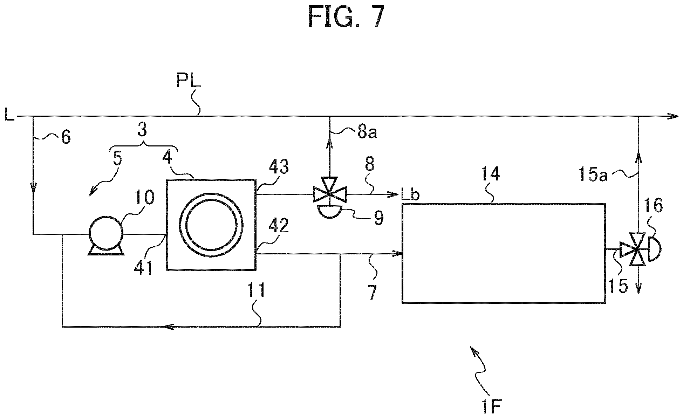

A system IF for preparing a sample solution for testing in FIG. 7 corresponds to an example in which the system 1E for preparing a sample solution for testing in FIG. 5 is connected to a test device. Specifically, a sample solution for testing can be directly supplied to the test device. One of the first segment and the second segment after separation in the hydrodynamic separation device can be returned to the manufacturing line PL, and the other one can be supplied as a sample solution for testing. In FIG. 7, the first segment is supplied to the test device. Further, the configuration is made so that the liquid is returned to the manufacturing line via the test device.

Specifically, the system IF for preparing a sample solution for testing includes a test device 14 and the flow channel 7 that connects the hydrodynamic separation device 4 to the test device 14. The flow channel 7 connects the outlet port 42 of the hydrodynamic separation device 4 and the test device 14 to each other, and the liquid discharged as the first segment is supplied to the test device 14 as a sample solution for testing. The system IF for preparing a sample solution for testing further includes a flow channel that connects the test device 14 to the manufacturing line PL. Specifically, a flow channel 15 for discharging the sample solution after testing from the test device is branched, and a return channel 15a is provided. At the branching point, a switching valve 16 is provided. Therefore, the sample solution after testing can be discharged to the outside of the system or can be returned to the manufacturing line PL in a selective manner, and a change therefore can be made by the switching valve 16.

As illustrated in FIG. 7, the hydrodynamic separation device and the test device is directly connected to each other through intermediation of a pipe, and thus contamination can be prevented from a location where the sample solution for testing is prepared from the undiluted sample solution to a location where the sample solution is supplied for testing. Thus, similarly, when the hydrodynamic separation device is connected to the test device in the system for preparing a sample solution for testing in each of FIG. 1 to FIG. 6, preparation of a sample solution for testing and a test can be performed in an aseptic state.

Through use of the system in FIG. 7, extraction of the undiluted sample solution from the manufacturing line and testing therefore can be performed sequentially. When no abnormality is found during testing, the solution derived from the undiluted sample solution can be returned to the manufacturing line. When abnormality is found, the switching valves 9 and 16 are controlled so that all the solution derived from the undiluted sample solution is discharged to the outside of the system. Further, the configuration may be made so that an on-off valve is provided to the supply channel 6 branched from the manufacturing line PL. With this, the undiluted sample solution may be extracted in a freely selective manner, which is suitable for conducting a regular test with intermittent extraction.

As shown in Expression given above, the De number is changed in accordance with the turning radius Rc of the curved flow channel and the dimension of the cross-section of the flow channel (the representative length D in Expression given above can be regarded as the width of the curved flow channel). Therefore, adjustment can be performed based on the design of the curved flow channel so as to obtain a suitable value for the De number. With this, the hydrodynamic separation device can perform concentration and separation of particles with satisfactory separation efficiency. Further, the flow rate of the liquid can be adjusted in accordance with the settings of any one of the width (radial cross-section) and the height of the cross-section of the curved flow channel. Thus, the hydrodynamic separation device may be configured based on the design of the curved flow channel so that separation of the particles can be performed at a desired flow rate. Therefore, the design of the curved flow channel may be changed as appropriate so that suitable separation is achieved in accordance with conditions of the separation target (dimension distribution of microorganisms, viscosity of a liquid, and the like). From a viewpoint of efficiency in separating the microorganism particles, it is suitable that the curved flow channel is a flow channel with a rectangular cross-section having an aspect ratio (width/height) of 10 or greater. The liquid is supplied to the curved flow channel described above at a flow rate of approximately 100 mL to 500 mL per minute. With this, separation progresses satisfactorily, and the microorganism particles can be concentrated and separated at efficiency of approximately five billion to twenty-five billion cells per minute. In this case, the microorganism particles having a particle diameter of approximately 10 µm or larger can be concentrated and isolated as the segment on the outer circumferential side. The relatively small microorganism particles having a particle diameter of approximately 5 µm or smaller are distributed in a ring-like shape, and the most part thereof is isolated as the segment on the inner circumferential side. With the design of the terminal end outlet of the curved flow channel (positions of the divided outlet ports), a size and separation accuracy of the particles contained in the segment on the outer circumferential side can be adjusted. Thus, the lower limit size of the microorganism particles concentrated in the segment on the outer circumferential side can be smaller than 10 µm. As illustrated in FIG. 5 to FIG. 7, the hydrodynamic separation step is repeated as required, thereby improving a concentration degree and separation accuracy of the particles.

A separation state of the particles in the hydrodynamic separation device (a size and an isolation ratio of the concentrated particles) differs depending on positions of the divided outlet ports. In general, a cross-sectional area ratio of the flow channel outlet is designed so that a division ratio (volume ratio) of the segment on the outer circumferential side/the segment on the inner is approximately 10/90 to 70/30, which is suitable for concentration and separation for the particles as described above. In the system for preparing a sample solution for testing in FIG. 1 to FIG. 7, the hydrodynamic separation device has the two outlet ports as the outlet of the curved flow channel, but may have three or more divided outlet ports. When the terminal end outlet is divided into three or more outlet ports, a configuration in which an amount of the isolated segment can be changed in accordance with a circumstance may be adopted.

The system for preparing a sample solution for testing described above is used, and thus sample solutions for testing can be prepared from various types of undiluted sample solutions. Specific examples that can be used as the undiluted sample solution include liquids such as a liquid raw material, a liquid intermediate, a liquid final product in manufacturing pharmaceutical and medical supplies and chemical products, a culture solution for microorganisms or cells, drinking water, water for manufacture and a food sample extracted form food. Examples of the water for manufacture include water for manufacturing medical and pharmaceutical products, water for manufacturing food and drink, and water for washing that is used in manufacturing industrial products. Examples of the liquid final product include a refreshing beverage, a lactic fermenting beverage, milk, beer, Japanese sake, and alcoholic beverages such as spirits. Examples of water for washing industrial products include water used in manufacturing process of an electronic substrate, a semiconductor, a precision apparatus, and medical equipment.

In the system for preparing a sample solution for testing according to the present disclosure, an impact on the components contained in the liquid is small during hydrodynamic separation. Thus, the system is applicable to a test for a substance that is easily deteriorated. For example, a glycoprotein such as an antibody contained in a liquid for culturing cells is a long chain polymer substance that is vulnerable. Even for such a substance, a sample solution for testing can be prepared through use of hydrodynamic separation. Specifically, through hydrodynamic separation, the cells contained in the culturing liquid are concentrated in the first segment, and are removed. Then the second segment can be subjected to testing and measurement. Therefore, the system for preparing a sample solution for testing in FIG. 4 is used, thereby suitably preparing a sample solution for testing.

Examples of the microorganism includes a procaryote (a eubacterium and an archaebacterium), a eucaryote (an alga, a protist, a fungus, and a myxomycete), and a virus. Specifically, examples of the procaryote include bacteria such as a colon bacillus, a hay bacillus, and Staphylococcus aureus, and hay bacilli such as actinomyces, cyanobacteria, and methanogenic bacteria. Examples of the eucaryote include algae such as spirogyra and wakame seaweed, protists such as a paramecium, and eumycetes such as mold, a yeast, a mushroom. Further, examples of the protists include amebas. Trochelminths such as a rotifer and microalgae such as Botryococcus braunii are also included as microorganisms.

Hydrodynamic separation is applicable to a liquid culture medium for culturing microorganisms. The liquid culture medium may be any liquid culture medium such as a synthetic medium, a semi-synthetic medium, and a natural medium, and may contain a nutrient, a differentiating agent for the purpose of testing (such as a pH indicator, an enzyme substrate, and a sugar), a selective agent for suppressing growth of microorganisms other than target microorganisms, and the like. A liquid culture medium having low viscosity is preferably used so that hydrodynamic separation efficiently progresses. Further, in a case of a microorganism particle such as a virus having a small particle diameter, a flocculation agent may be added to a liquid culture medium so as to form a particle having a size that facilitates hydrodynamic separation. Examples of the flocculation agent include a polymer flocculation agent and an inorganic flocculation agent such as polyaluminum chloride, aluminum sulfate, and polysilica iron.

When the test device 14 is a test device for microorganisms, the test device 14 may include means for measuring at least one of the number of microorganism particles, particle diameter distribution, and a survival rate of the microorganisms. With a test device performing an optical analysis, a sequential and efficient test can be conducted. Further, measurement performed by the test device 14 may involve operations such as culture, observation with a microscope, staining or light emission with a reagent, fluorescence detection, an electrophoresis, and absorption spectrum measurement through use of an electromagnetic wave such as an infrared spectrum. Further, an operation utilizing an indicator such as a marker selectively reacting to a specific component, structure, gene may be performed. Measurement for microorganisms may be performed by an operation such as an optical analysis through flow cytometry. Examples of the devices capable of performing those operations include a flow cytometer (product name: Attune NxT) available from Thermo Fisher Scientific Inc., a cell analyzer (product name: Vi-Cell) available from Beckman Coulter Inc., and a plate reader (product name: ViewLux) available from PerkinElmer Inc.

The sample solution for testing may be used to culture the microorganisms contained in the liquid. The microorganisms may be cultured in accordance with a usual method under culture conditions that have been known to be suitable for the microorganisms. A method suitable for the microorganisms to be cultured may be selected and used as appropriate. In general, a selective enrichment medium or a selective separation medium prescribed for increasing a specific strain type is suitably used. The culture medium may be selected and used from commercially available liquid culture media as appropriate, or may be produced in accordance with a known prescription through use of a nutrient, purified water, and the like. A differentiating agent for the purpose of testing (such as a pH indicator, an enzyme substrate, and a sugar), a selective agent for suppressing growth of microorganisms other than target microorganisms, and the like may be added as required.

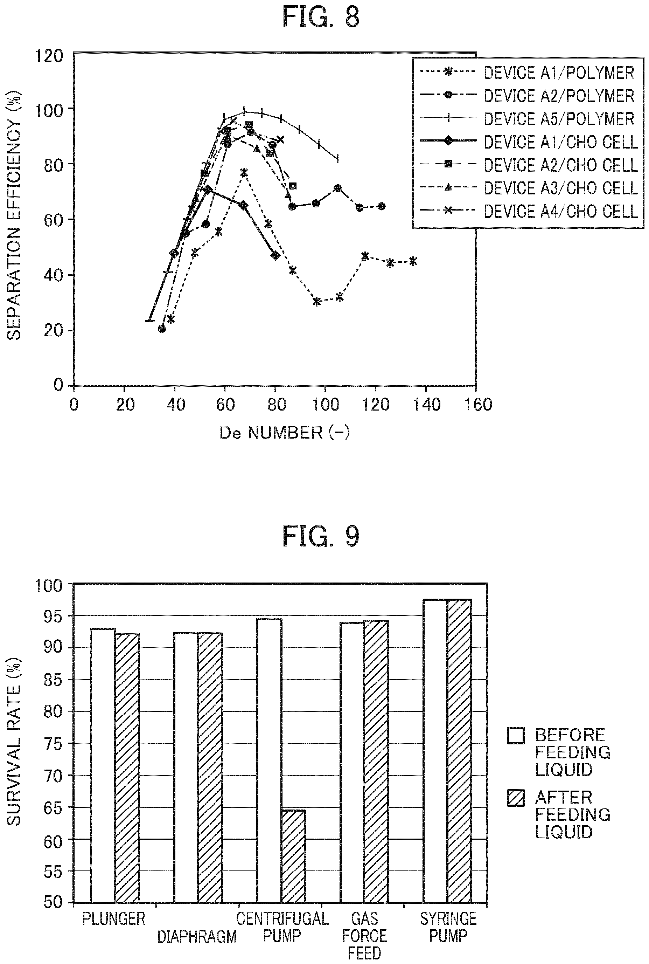

FIG. 8 is a graph showing results of examination on a relationship between separation efficiency and a De number in the hydrodynamic separation device. Specifically, any one of five types of hydrodynamic separation devices (devices A1 to A5) having different flow channel dimensions and any one of separation targets being polymer particles (styrene-divinylbenzene polymer particles) and Chinese hamster ovary cells (CHO cells) were used, and separation was performed in the hydrodynamic separation devices. Any one of the separation targets had a particle diameter average falling within a range from 14 µm to 18 µm. Separation efficiency is a value obtained by calculating [1-(x/X)]×100 (%), where, in the expression, X denotes a concentration of the separation target contained in the liquid before separation, and x denotes a concentration of the separation target contained in the segment on the inner circumferential side after separation. Any one of the separation targets had narrow particle diameter distribution. Thus, evaluation was given by regarding separation efficiency as 100% under a state in which the total amount of particles or cells concentrated in the segment on the outer circumferential side. As understood from the graph, separation efficiency was the highest with the De number around 70 in both the cases of the polymer particles and the animal cells. In general, high separation efficiency can be achieved under a condition where the De number falls within a range from 50 to 80. Therefore, it can be understood that, due to hydrodynamic separation, the particles can be concentrated and separated regardless of a particle composition.

FIG. 9 shows results of examination on how a pump type, which fed a liquid culture medium to a hydrodynamic separation device, affected a survival rate of animal cells. The liquid culture medium for culturing cells was supplied to the hydrodynamic separation device at an ejection pressure of 0.3 MPa through use of a pump. Two segments containing liquid culture media discharged from the separation device were collectively gathered, and a small amount thereof was taken as samples. The results were obtained by measuring a survival rate of cells in each sample (%, a rate of living cells in the total cell numbers) through use of a cell analyzer (available from Beckman Coulter Inc., product name: Vi-Cell). "Gas force feed" in the graph indicates a mode in which the liquid culture medium was pressurized and fed by a compressed air from a pressure tank to which a compressor was attached, and can be categorized as a positive displacement pump. From FIG. 9, it can be understood that the cells were heavily damaged by a centrifugal pump and that reduction in survival rate was prevented with the other pumps categorized as a positive displacement pump. The microorganism particles had higher durability than the animal cells, and hence were not significantly affected by the pump types as in FIG. 9. Nevertheless, in a case where durability of the concentrated particles is unclear, a pump may be selected as appropriate.

As described above, through use of the hydrodynamic separation technique, the particles contained in the undiluted sample solution can be concentrated and separated, and thus the sample solution for testing can be prepared. At the time of preparation, damage on the solid matter particles and an impact on the microorganisms can be prevented. Hydrodynamic separation can promote concentration and separation for particle components much more efficiently than centrifugal separation, and can perform concentration and separation with a higher survival rate without damaging microorganisms as compared to filter separation and the like. Further, an expensive component or a rear substance can be recovered. The liquid is supplied to the curved flow channel at a relatively high speed during hydrodynamic separation. Thus, the system for preparing a sample solution for testing according to the present disclosure has high processing capacity of performing concentration and separation for particles, and a sample solution for testing can be prepared efficiently. Further, the present disclosure is sufficiently applicable to quality control and safety control in manufacturing products.

### Example 1

With reference to a particle separator described in Patent Literature 1 given above, a resin molded body having a flat plate-like shape was produced. Inside the molded body, an arc-shaped curved flow channel (a turning diameter: approximately 115 mm, a radial cross-section of a flow channel: a rectangular shape, a flow channel width: approximately 3 mm) was formed. The molded body was used as a flow channel unit, and a hydrodynamic separation device was configured. A syringe pump (that pressurized and fed the liquid by pushing and pulling a plunger repeatedly) was used as the pump 10 for supplying the liquid, and the hydrodynamic separation device described above was used. In this manner, the system 1 for preparing a sample solution for testing in FIG. 3 was configured. Here, the return channel 8a was not provided, each of the first segment and the second segment was recovered.

Yeast were added and dispersed in water. A yeast aqueous liquid containing yeast particles was thus produced as an undiluted sample solution, and was stored in the container 2. Through use of the syringe pump, the yeast aqueous liquid in the container 2 was pressurized and fed to the curved flow channel of the hydrodynamic separation device at a constant flow rate of 110 mL/min. The first segment on the outer circumferential side and the second segment on the inner circumferential side that are discharged from the outlet ports 42 and 43 of the hydrodynamic separation device, respectively, were recovered in recovery containers. For each of the undiluted sample solution and the first segment, the number of yeast contained in the liquid and a survival rate (a rate of living yeast in the total number of yeast) were measured through use of an image type particle measurement device (available from Beckman Coulter Inc., product name: Vi-Cell). This operation and measurement were performed six times. The results obtained in each time are shown in Table 1. Note that a concentration rate in Table 1 is a yeast concentration in the first segment, and is expressed as a percentage (%). The concentration rate was obtained while regarding the number of yeast particles per unit volume of the liquid as a yeast concentration and regarding a yeast concentration in the undiluted sample solution as a reference.

**[Table 1]**

| | Concentration rate [%] | Survival rate of yeast [%] | |
|---|---|---|---|
| | | Undiluted Sample solution | First segment |
| First time | 115 | 83.8 | 84.3 |
| Second time | 125 | 83.8 | 86.5 |
| Third time | 130 | 83.8 | 83.9 |
| Fourth time | 106 | 83.8 | 84.0 |
| Fifth time | 121 | 83.8 | 84.4 |
| Sixth time | 127 | 83.8 | 87.5 |

As understood from Table 1, in any one of the six operations, the yeast were concentrated in the first segment. Significant reduction of a survival rate of the yeast was not confirmed before and after hydrodynamic separation. Thus, it is concluded that a separation operation caused little damage on the yeast. Further, a survival rate of the yeast in the first segment was significantly increased as compared to the yeast aqueous liquid before separation. The survival rate was increased because a rate of the relatively small particles contained in the first segment was reduced, and thus a rate of dead fungus bodies, debris, and impurities was reduced. Therefore, it is clear that the first segment in which the yeast were concentrated was able to be recovered through hydrodynamic separation without reducing a survival rate of the yeast and be supplied as a sample solution for testing.

### Example 2

A suitable amount of an amino acid (L-alanyl-L-glutamine) and a suitable amount of an antibiotic (penicillin, streptomycin, amphotericin B) were added to a liquid culture medium of 1.5 L (available from GE Healthcare, product name: SH30934.01 HyCell CHO Medium). CHO cell strains (ATCC CRL-12445 Cricetulus griseus/CHO DP-12 clone#1932) were cultured in the liquid culture medium while maintaining the temperature from 36.5 degrees Celsius to 37.0 degrees Celsius. During culture, a pH value of the liquid culture medium was controlled so that the pH value was not reduced under 6.70. The culture liquid thus obtained contained an IgG antibody secreted by the cells, and a concentration thereof was measured.

Similarly to Example 1, the system for preparing a sample solution for testing was configured, and the cell culture liquid obtained as described above was stored as the undiluted sample solution in the container 2. Through use of the syringe pump, the cell culture liquid in the container 2 was pressurized and fed to the curved flow channel of the hydrodynamic separation device at a constant flow rate. The first segment on the outer circumferential side and the second segment on the inner circumferential side that are discharged from the outlet ports 42 and 43 of the hydrodynamic separation device, respectively, were recovered in recovery containers. For the second segment, the number of cells contained in the liquid was counted through use of an image type particle measurement device (available from Beckman Coulter Inc., product name: Vi-Cell). Further, a concentration of the IgG antibody contained in the second segment was measured.

This operation and measurement were performed three times. The results obtained in each time are shown in Table 2. Note that the cell separation efficiency in Table 2 is a value obtained by calculating [1-(x/X)]×100 (%), where, in the expression, X denotes a cell concentration in the undiluted sample solution, and x denotes a cell concentration of the second segment (the segment on the inner circumferential side) after separation.

**[Table 2]**

| | Cell separation efficiency [%] | Concentration of IgG antibody [mg/L] | |
|---|---|---|---|
| | | Undiluted Sample solution | Second segment |
| First time | 92.0 | 14.0 | 14.8 |
| Second time | 92.3 | 19.0 | 19.5 |
| Third time | 92.6 | 26.0 | 25.7 |

As understood from Table 2, in any one of the three operations, the separation efficiency was 90% or higher, and the cells were concentrated in the first segment. Further, a significant change in antibiotic concentration was not found before or after hydrodynamic separation. Based on this fact, even a vulnerable long chain protein such as an antibody protein can be supplied for testing without causing composition changes. In other words, an analysis on components in a liquid such as a microorganism test can be conducted without changing the components in the liquid. With this, quality of the liquid can be confirmed. Therefore, by preparing a sample solution for testing through utilization of hydrodynamic separation, degradation of analysis quality, which is caused by contamination with solid components, can be prevented, and a highly accurate test can be conducted.

### INDUSTRIAL APPLICABILITY

A sample solution for testing can be prepared efficiently by concentrating and isolating particles contained in a liquid without affecting components contained in the liquid. The sample solution for testing can be used for various tests in manufacturing medical and pharmaceutical products, or food and drink, and quality control and safety control of a manufactured product can be performed efficiently. This utilization contributes to economic efficiency and quality improvement.

## Claims

1. A system for preparing a sample solution for testing, the system being configured to prepare a sample solution for testing from an undiluted sample solution and comprising a microorganism separation device, the microorganism separation device comprising:
a hydrodynamic separation device including a curved flow channel having a rectangular cross-section and being configured to separate a liquid into a first segment and a second segment through use of a vortex flow generated in the liquid flowing through the curved flow channel; and
a liquid feeding unit configured to supply the undiluted sample solution to the hydrodynamic separation device, wherein
with the hydrodynamic separation device, particles contained in the undiluted sample solution are concentrated in the first segment.

2. The system for preparing a sample solution for testing according to claim 1, wherein
the undiluted sample solution comprises a liquid raw material, a liquid intermediate, and a liquid final product in manufacturing food and drink or pharmaceutical products.

3. The system for preparing a sample solution for testing according to claim 1 or 2, wherein
the liquid feeding unit includes:
a supply channel being branched from a manufacturing line and connected to the hydrodynamic separation device; and
a pump configured to apply, to the undiluted sample solution, a hydrodynamic pressure for supplying the undiluted sample solution to the hydrodynamic separation device, and
part of a liquid raw material, a liquid intermediate, or a liquid final product that flows through a manufacturing line is supplied as the undiluted sample solution to the hydrodynamic separation device.

4. The system for preparing a sample solution for testing according to claim 3, wherein
the liquid feeding unit further includes a return channel that returns one of the first segment and the second segment to the manufacturing line.

5. The system for preparing a sample solution for testing according to claim 1 or 2, wherein
the liquid feeding unit includes:
a container configured to store the undiluted sample solution;
a supply channel configured to connect the container to the hydrodynamic separation device; and
a pump configured to apply, to the undiluted sample solution, a hydrodynamic pressure for supplying the undiluted sample solution to the hydrodynamic separation device.

6. The system for preparing a sample solution for testing according to claim 5, wherein
the liquid feeding unit further includes a return channel configured to return one of the first segment and the second segment to the container.

7. The system for preparing a sample solution for testing according to claim 4, wherein
the liquid feeding unit further includes a circulation channel configured to cause a part of another one of the first segment and the second segment to circulate to the supply channel.

8. The system for preparing a sample solution for testing according to claim 7, further comprising:
an additional hydrodynamic separation device, wherein
the liquid feeding unit further includes a flow channel for supplying, to the additional hydrodynamic separation device, a remaining part of another one of the first segment and the second segment after separation in the hydrodynamic separation device.

9. The system for preparing a sample solution for testing according to claim 4, further comprising:
a test device;
a flow channel configured to connect the hydrodynamic separation device to the test device; and
a flow channel configured to connect the test device to the manufacturing line, wherein
another one of the first segment and the second segment is returned to the manufacturing line via the test device.

10. The system for preparing a sample solution for testing according to claim 4 or 6, wherein
the particles are microorganisms of at least one type selected from a virus, a eubacterium, an archaebacterium, a fungus, a myxomycete, an alga, and a protist,
the first segment is supplied as a sample solution for testing, and
the return channel returns the second segment from the hydrodynamic separation device.

11. The system for preparing a sample solution for testing according to claim 4 or 6, wherein
the particles are particle-like solid matters larger than microorganisms,
the second segment is supplied as a sample solution for testing, and
the first segment is returned through the return channel or is discharged to an outside of the system.

12. A method for preparing a sample solution for testing, the method being performed to prepare a sample solution for testing from an undiluted sample solution and comprising microorganism separation processing, the microorganism separation processing comprising:
a hydrodynamic separation step of separating a liquid into a first segment and a second segment through use of a vortex flow generated in the liquid flowing through a curved flow channel having a rectangular cross-section; and
a liquid feeding step of feeding the undiluted sample solution to the hydrodynamic separation step, wherein
in the hydrodynamic separation step, particles contained in the undiluted sample solution are concentrated in the first segment.
